# EUROPEAN PATENT APPLICATION

(11) **EP 3 566 954 A1**
(43) Date of publication of application: **13.11.2019**
(21) Application number: 17917216.8
(22) Date of filing: 29.11.2017
(51) Int. Cl.: B65B 3/04

(54) **EXTERNAL FEEDING SYSTEM**

(30) Priority: 15.09.2017 CN 201721210075 U
(71) Applicant: Amsino Medical (Shanghai) Co., Ltd., Shanghai 201613 (CN)
(72) Inventor: LI, Zhongze, Shanghai 201803 (CN); LEE, Richard, Pomona, CA 91768 (US)
(74) Representative: Hoffmann Eitle
(86) International application number: PCT/CN2017/113469
(87) International publication number: WO 2019/052016

(57) **Abstract**

Disclosed is a feeding system comprising a receiving container and a feeding container; the receiving container comprises a receiving container body, a top cover, and a feeding connector; the feeding connector is connected with the top cover, and the feeding connector is provided with a feeding channel; the feeding container comprises a feeding container body and a neck portion; a lower end of the neck portion has an outlet; a side of the neck is provided with a guide slot; a sidewall of the feeding channel is provided with a guide lug mating with the guide slot; the guide slot is shaped in a way of guiding the feeding container to move downward from an initial position when the feeding container rotates in a predetermined direction; the seal destruction portion of the feeding connector destructs the seal of the outlet while the feeding container is moving downward. According to the present invention, it is possible to feed a material into the receiving container without opening the top cover of the receiving container.

## Description

### Field of the Invention

The present disclosure relates to a system for feeding a material into a container from outside.

### Background of the Invention

At present, various liquid medical waste generated during clinical treatment in hospitals is usually sucked into a liquid waste collection tank by a negative pressure suction device. The liquid waste collection tank comprises a liquid waste collection bag, a tank body and a top cover. The liquid waste collection bag is disposed in the tank body. The top cover covers at a mouth of the tank body and is sealingly connected with a top portion of the liquid waste collection bag. On the top cover is usually provided a liquid waste outlet and a suction port communicated with the liquid waste collection bag.

When the liquid waste collection bag is filled with liquid waste, the liquid waste needs to be disposed. As the liquid medical waste contains a lot of bacteria, it is difficult to purify the liquid waste in an ordinary disposing manner of drain water. To prevent the liquid medical waste from being improperly drained into a sewage system and causing pollution to the environment, it is a commonly-used disposing manner to feed a solidifier into the liquid waste collection bag after the liquid waste collection bag is full of liquid waste, and then to dispose the coagulated liquid waste. At present, upon feeding the solidifier into the liquid waste collection bag, the doctors must open the top cover. Since liquid medical waste contains a lot of bacteria, the manner of feeding by opening the top cover not only causes inconvenience to the doctor's operations but also might cause cross infection due to improper operations.

### Summary of the Invention

The present invention is directed to provide a feeding system which is simple in structure, convenient for operation and capable of feeding material into a receiving container without opening the top cover of the receiving container.

A technical solution employed by the present invention is: a feeding system comprising a receiving container and a feeding container; the receiving container comprises a receiving container body, a top cover covering an upper end of the receiving container body, and a feeding connector; the feeding connector is connected with the top cover, and the feeding connector is provided with a feeding channel communicated with the receiving container body; the feeding container comprises a feeding container body and a neck portion; an upper end of the neck portion is connected with the feeding container body, and a lower end of the neck portion has an outlet; the feeding container is provided with a seal which seals the outlet; the neck portion of the feeding container is rotatably mounted in the feeding channel of the feeding connector; a side of the neck is provided with a guide slot; a sidewall of the feeding channel is provided with a guide lug mating with the guide slot; the guide lug extends into the guide slot; the guide slot is shaped in a way of guiding the feeding container to move downward from an initial position when the feeding container rotates in a predetermined direction due to the action of an external force; the feeding connector is provided with a seal destruction portion, and the seal destruction portion is used to destruct the seal while the feeding container moves downward, so that the material in the feeding container flows through the outlet of the neck portion into the receiving container body.

Advantages of the present invention are as follows:
1. The feeding system according to an embodiment of the present invention has less components whose structures and shapes are uncomplicated, and thus those components are easy to manufacture. When the material needs to be fed, as long as the feeding container is rotated in a predetermined direction, the feeding container will move downward under the guidance of the guide slot and the seal destruction portion will destruct the seal with the movement of the feeding container, such that the feeding can be operated in a convenient manner;
2. The outside surface of the neck portion of the feeding container of the feeding system according to an embodiment of the present disclosure sealingly engages with the sidewall of the feeding channel of the feeding connector. Thus, the embodiment of the present invention can ensure that the feeding system retains sealed when the liquid waste is collected under a negative pressure and material can be fed to the liquid waste can be operated when the liquid waste in the receiving container 1 is in a sealed and isolated state from the external, thereby reducing the risk of the secondary pollution and improving safety of the feeding operation.

### Brief Description of Drawings

The present invention will be further described with reference to figures.
Fig. 1 shows a schematic diagram of an appearance of a feeding system according to an embodiment of the present invention.
Fig. 2 shows a partially-enlarged schematic diagram of a feeding system according to an embodiment of the present invention.
Fig. 3 through Fig. 6 show a schematic diagram (local cross-sectional view) of a process in which a feeding container moves downward from an initial position according to an embodiment of the present invention.
Fig. 7 shows a perspective view of a feeding container according to an embodiment of the present invention.
Fig. 8 and Fig. 9 show an appearance of a feeding container according to an embodiment of the present invention from different perspectives respectively.
Fig. 10 shows a perspective view of a feeding connector according to an embodiment of the present invention, wherein a seal destruction portion of the feeding connector has a first structure.
Fig. 11 shows a sectional view of a feeding connector according to an embodiment of the present invention, wherein a seal destruction portion of the feeding connector has a first structure.
Fig. 12 shows a perspective view of a feeding connector according to an embodiment of the present invention, wherein a seal destruction portion of the feeding connector has a second structure different from the first structure.
Fig. 13 shows a sectional view of a feeding connector according to an embodiment of the present invention, wherein a seal destruction portion of the feeding connector has a second structure different from the first structure.

### Detailed Description of Preferred Embodiments

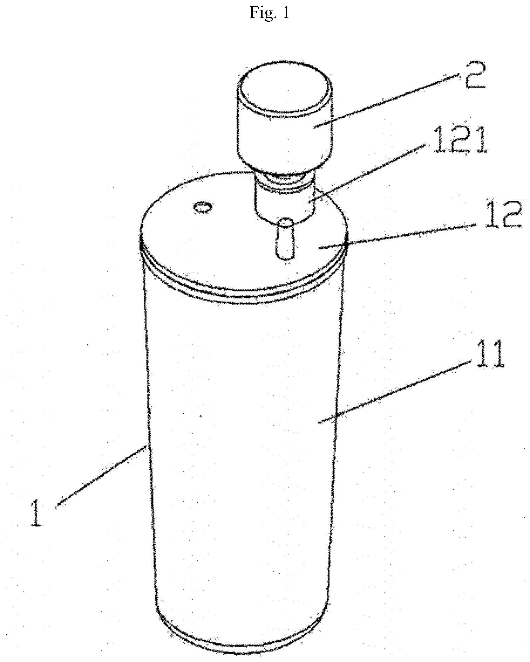

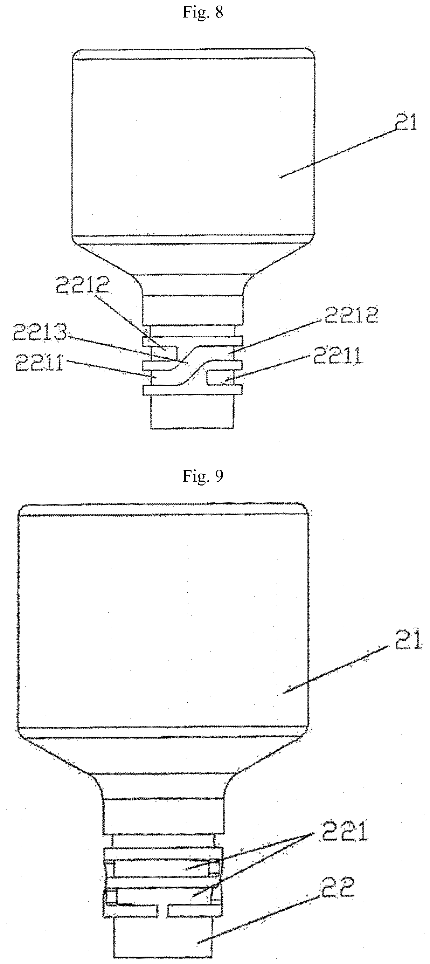

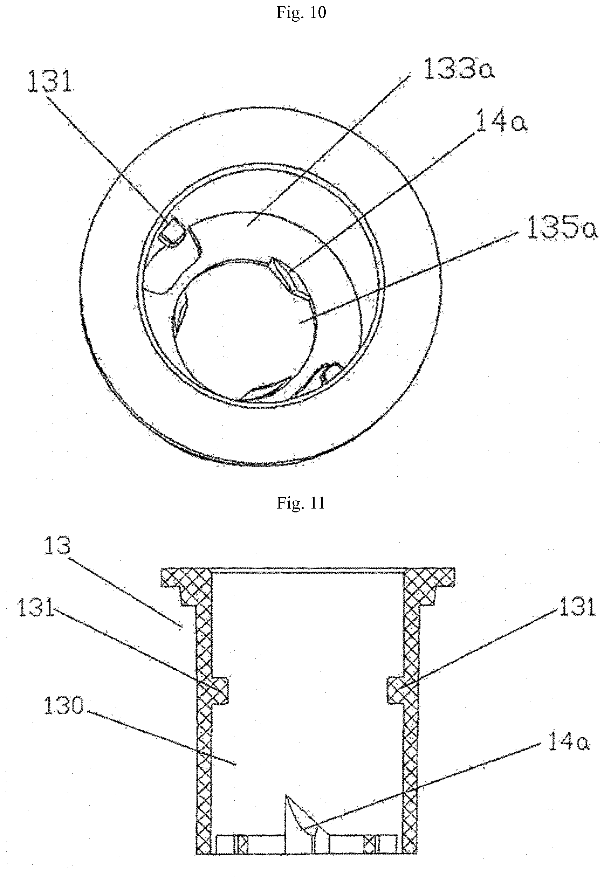

Reference is made to Fig. 1 through Fig. 11. A feeding system according to an embodiment of the present invention comprises a receiving container 1 and a feeding container 2.

The receiving container 1 comprises a receiving container body 11, a top cover 12 covering an upper end of the receiving container body, and a feeding connector 13. The feeding connector 13 is substantially tubular, the feeding connector 13 is connected with the top cover 12, and the feeding connector 13 is provided with a feeding channel 130 communicated with the receiving container body 11.

The feeding container 2 comprises a feeding container body 21 and a neck portion 22. An upper end of the neck portion 22 is connected with the feeding container body 21, and a lower end of the neck portion 22 has an outlet 23. The feeding container 2 is provided with a seal 24 which seals the outlet 23. The seal 24 preferably employs a film-like seal which for example may be made of an aluminum foil film. The neck portion 22 of the feeding container 2 is rotatably mounted in the feeding channel 130 of the feeding connector 13. A side of the neck 22 is provided with a guide slot 221. A sidewall of the feeding channel 130 is provided with a guide lug 131 mating with the guide slot 221. The guide lug 131 extends into the guide slot 221. The guide slot 221 is shaped in a way of guiding the feeding container 2 to move downward from an initial position when the feeding container 2 rotates in a predetermined direction due to the action of an external force. The number of guide lug 131 may be one or more. When the number of the guide lugs 131 is plural, the plurality of guide lugs 131 mate with the plurality of guide slots 221 in one to one correspondence.

In the present embodiment, the guide slot 221 comprises a first displacement slot 2211, a skewed slot 2213 and a second displacement slot 2212. The first displacement slot 2211 is parallel to the second displacement shot 2212, an end of the skewed slot 2213 is connected with the first displacement slot 2211, and the other end of the skewed slot 2213 is connected with the second displacement slot 2212.

Preferably, a plurality of first displacement slots 2211 mating with the plurality of guide lugs 131 respectively are all located at a first circumference, and a plurality of second guide slots 2212 corresponding to the plurality of guide lugs 131 are all located at a second circumference. In the present embodiment, the number of the guide lugs 131 is two.

The feeding connector 13 is provided with a seal destruction portion. The seal destruction portion is used to destruct the seal 24 while the feeding container 2 is moving downward, so that the material in the feeding container 2 flows into the receiving container body 11 through the outlet 23 of the neck portion 22.

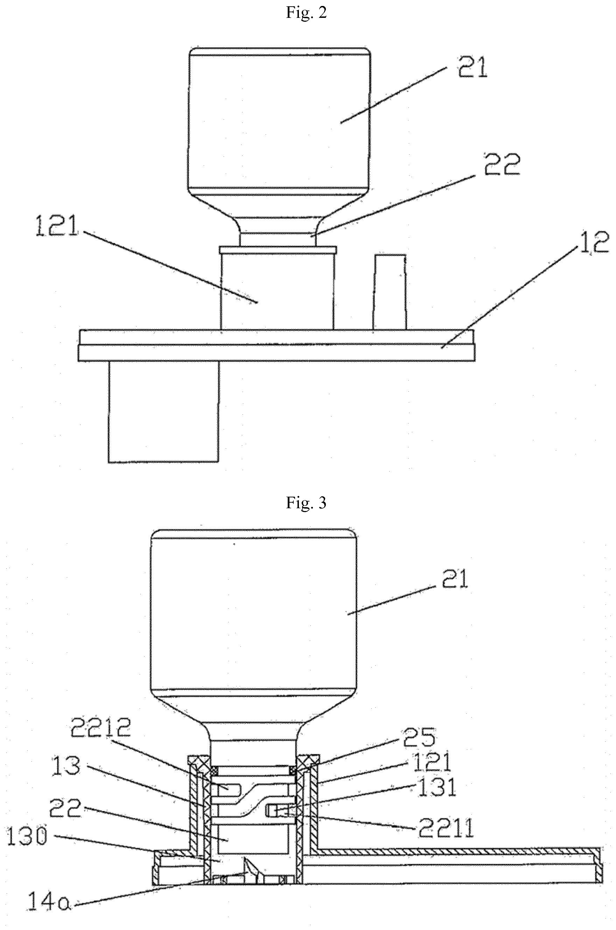

In the example shown in Fig. 3 through Fig. 11, the seal destruction portion is comprised of a plurality of splitting portions 14a which split the seal 24. In the figures, the feeding connector 13 is provided with a base plate 133a, a circumference of which is connected with the sidewall of the feeding channel 130. The base plate 13a is provided with a central through hole 135a, and a bottom end of the plurality of splitting portions 14a are connected with a wall of the central through hole 135a.

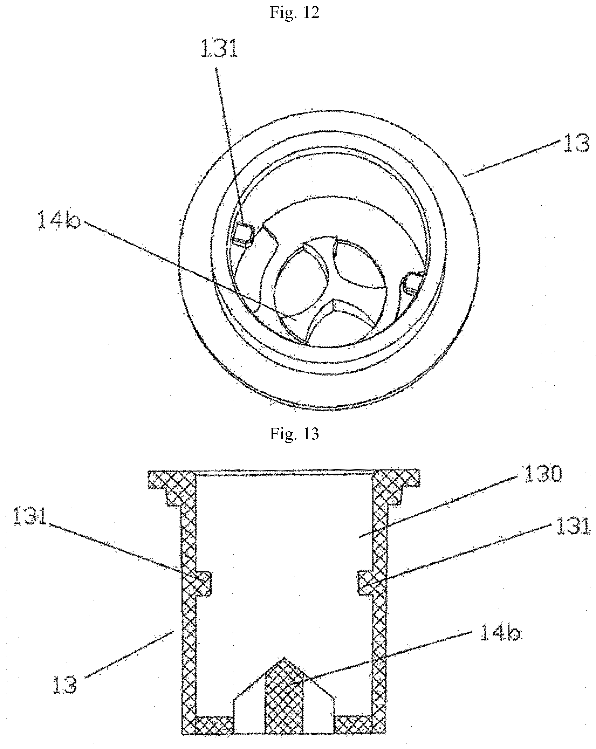

In other embodiments, as shown in Fig. 12 and Fig. 13, the seal destruction portion may be comprised of a hollowed tapered portion 14b, and a bottom of the tapered portion 14b is connected with the sidewall of the feeding channel 130 of the feeding connector 13.

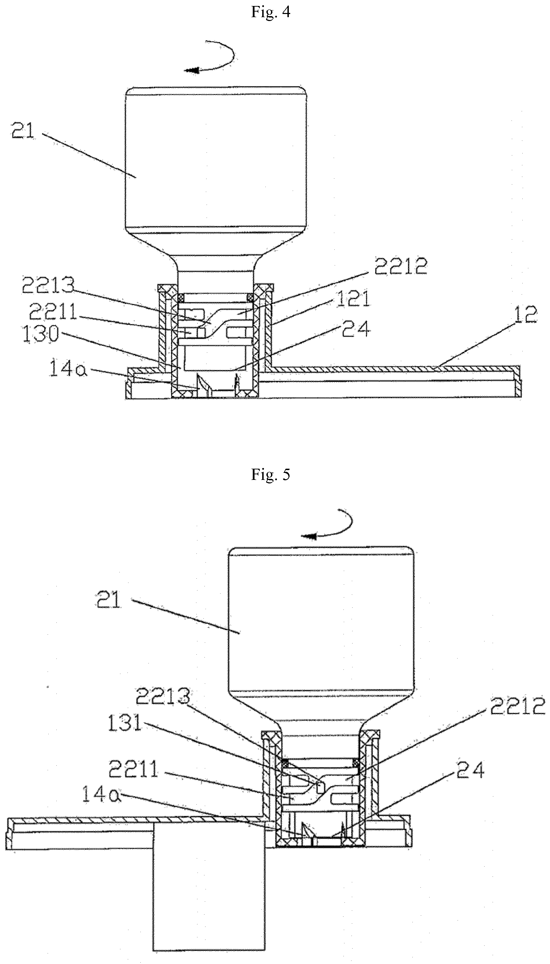

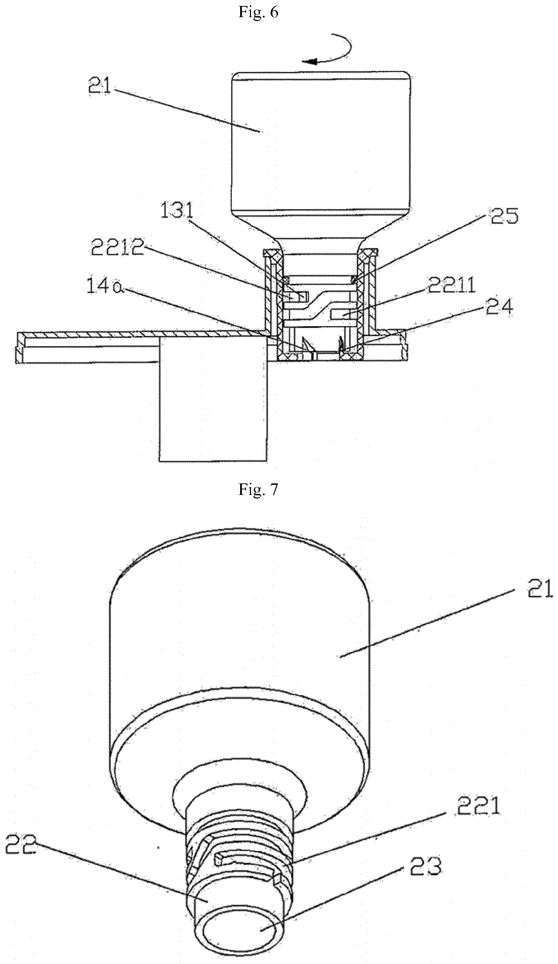

Fig. 3 through Fig. 6 show a working process in which the seal destruction portion destructs the seal 24. In Fig. 3, the feeding container 2 is at an initial position. At this time, the two guide lugs 131 are respectively located in the first displacement slot 2211 corresponding thereto, and the splitting portion 14a does not contact the seal 24. Since the two guide lugs 131 are spaced apart by 180° , the figures can only display one of the guide lugs 131. As shown in Fig. 4, when the feeding container 2 rotates in a predetermined direction (clockwise direction in Fig. 4) due to the action of an external force, the two guide lugs 131 respectively move along the first displacement shots 2211 to an end of the corresponding skewed slots 2213 at first, whereupon the splitting portion 14a still does not contact the seal 24. As shown in Fig. 5, the feeding container 2 continues to rotate in the clockwise direction, and the two guide lugs 131 move upward along the corresponding skewed slots 2213, i.e., the feeding container 2 moves downward. In this process, the plurality of splitting portions 14a contact and split the seal 24, such that the material in the feeding container 2 flows through the outlet 23 of the neck portion 22 into the receiving container body 11. If the seal destruction portion is comprised of the hollowed tapered portion 14b, a top end of the tapered portion 14b punctures the seal 24 while the two guide lugs 131 is moving upward along the corresponding skewed slots 2213. As shown in Fig. 6, as the feeding container 2 continues to rotate, the two guide lugs 131 move from the other end of the corresponding skewed slots 2213 into the second displacement slot 2212, whereupon the feeding container 2 will not move downward any longer.

In a specific application, the receiving container body 11 of the feeding system according to an embodiment of the present invention is a liquid waste collection bag, and the top cover 12 is sealingly connected with a top portion of the liquid waste collection bag. The top cover 12 is provided with a connector pipe 121 running through the top cover and extending upward. The feeding connector 13 is inserted in the connector pipe 121, and a periphery of the upper end of the feeding connector 13 is sealingly connected with the upper end of the connector pipe 121. The feeding container 2 is a feeding bottle, and the material in the feeding container 2 is a solidifier. To ensure that the feeding system retains sealed when the liquid waste is collected under a negative pressure and material can be fed to the liquid waste when the liquid waste in the receiving container 1 is in a sealed and isolated state from the external, thereby reducing the risk of the secondary pollution and improving safety of the feeding operation, the outside surface of the neck portion 22 of the feeding container 2 sealingly engages with the sidewall of the feeding channel 130 of the feeding connector 13. In the present embodiment, a sealing ring 25 is provided around the outside surface of the neck portion 22, and the sealing ring 25 sealingly abuts against the sidewall of the feeding channel 130. It can be seen from Fig. 3 through Fig. 6 that in the whole rotation process and downward movement process of the feeding container, the sealing ring 25 is in a state of sealingly abutting against the sidewall of the feeding channel 130.

### Industrial applicability

The feeding system according to an embodiment of the present invention has less components whose structures and shapes are uncomplicated, and thus those components are easy to manufacture. When the material needs to be fed, as long as the feeding container is rotated in a predetermined direction, the feeding container will move downward under the guidance of the guide slot and the seal destruction portion will destruct the seal with the movement of the feeding container, such that the feeding can be operated in a convenient manner.

## Claims

1. A feeding system, comprising a receiving container and a feeding container; the receiving container comprises a receiving container body, a top cover covering an upper end of the receiving container body, and a feeding connector; the feeding connector is connected with the top cover, and the feeding connector is provided with a feeding channel communicated with the receiving container body; the feeding container comprises a feeding container body and a neck portion, an upper end of the neck portion is connected with the feeding container body, and a lower end of the neck portion has an outlet; wherein,
the feeding container is provided with a seal which seals the outlet; the neck portion of the feeding container is rotatably mounted in the feeding channel of the feeding connector; a side of the neck is provided with a guide slot; a sidewall of the feeding channel is provided with a guide lug mating with the guide slot, and the guide lug extends into the guide slot; the guide slot is shaped in a way of guiding the feeding container to move downward from an initial position when the feeding container rotates in a predetermined direction due to the action of an external force;
the feeding connector is provided with a seal destruction portion, and the seal destruction portion is configured to destruct the seal while the feeding container is moving downward, such that a material in the feeding container flows through the outlet of the neck portion into the receiving container body.

2. The feeding system according to claim 1, wherein the guide slot comprises a first displacement slot, a skewed slot and a second displacement slot; the first displacement slot is parallel to the second displacement shot, an end of the skewed slot is connected with the first displacement slot, and the other end of the skewed slot is connected with the second displacement slot.

3. The feeding system according to claim 2, wherein the number of the guide lugs is plural, and the plurality of guide lugs mate with the plurality of guide slots in one to one correspondence;
a plurality of first displacement slots mating with the plurality of guide lugs respectively are all located at a first circumference, and a plurality of second guide slots corresponding to the plurality of guide lugs are all located at a second circumference.

4. The feeding system according to claim 3, wherein the number of the guide lugs is two.

5. The feeding system according to claim 1 or 2 or 3, wherein the seal destruction portion is comprised of a plurality of splitting portions configured to split the seal.

6. The feeding system according to claim 1 or 2 or 3, wherein the seal destruction portion is comprised of a hollowed tapered portion, and a bottom of the tapered portion is connected with a wall of the feeding channel of the feeding connector.

7. The feeding system according to claim 1 or 2 or 3, wherein an outside surface of the neck portion sealingly engages with the sidewall of the feeding channel of the feeding connector.

8. The feeding system according to claim 7, wherein a sealing ring is provided around the outside surface of the neck portion, and the sealing ring sealingly abuts against the sidewall of the feeding channel.

9. The feeding system according to claim 7, wherein the receiving container body is a liquid waste collection bag, and the top cover is sealingly connected with a top portion of the liquid waste collection bag; the top cover is provided with a connector pipe running through the top cover and extending upward; the feeding connector is inserted in the connector pipe, and a periphery of the upper end of the feeding connector is sealingly connected with an upper end of the connector pipe;
the feeding container is a feeding bottle.

10. The feeding system according to claim 1, wherein the seal is a film-like seal.
